(19) Europäisches Patentamt
European Patent Office
Office européen des brevets

(11) Publication number: 0 079 940 B1

(12) **EUROPEAN PATENT SPECIFICATION**

(45) Date of publication of patent specification: **24.08.88**

(51) Int. Cl.$^4$: **A 61 K 6/08**

(21) Application number: **82902043.7**

(22) Date of filing: **20.05.82**

(86) International application number: **PCT/US82/00695**

(87) International publication number: **WO 82/04259 09.12.82 Gazette 82/29**

# (54) FLUORINE CONTAINING DENTAL MATERIALS.

(30) Priority: **01.06.81 US 269129**

(43) Date of publication of application: **01.06.83 Bulletin 83/22**

(45) Publication of the grant of the patent: **24.08.88 Bulletin 88/34**

(84) Designated Contracting States: **AT CH DE FR GB LI NL SE**

(56) References cited:
US-A-2 824 083
US-A-2 980 733
US-A-3 159 470
US-A-3 327 016

CHEMICAL ABSTRACTS, vol. 85, no. 18, November 1, 1976, abstract 130556b, Columbus, Ohio, US

(73) Proprietor: **Kerr Manufacturing Company (a Delaware corporation)**
**28200 Wick Road Box 455**
**Romulus, Michigan 48174 (US)**

(72) Inventor: **O'CONNELL, John J.**
**14671 Cheshire Place**
**Tustin, CA 92680 (US)**
Inventor: **KWAN, Stephen Chiamin**
**301 Sunkist Lane**
**Los Altos, CA 94022 (US)**

(74) Representative: **Oliver, Roy Edward et al**
**POLLAK MERCER & TENCH High Holborn House**
**52-54 High Holborn**
**London WC1V 6RY (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 079 940 B1

Courier Press, Leamington Spa, England.

**Description**

Background of the invention

It is established that dental silicate cements containing fluoride are therapeutic in preventing secondary caries and reducing plaque formation. Present acrylic denture base material, restorative materials and adhesives have been shown to be sites of bacterial and plaque accumulation, which can be a precursor of irritation to soft tissues and caries attack on remaining natural dentition. The release of fluoride ion from these dental restorative materials occurs either by surface release, or by dissolution of the fluorine-containing additives or the dental restorative material itself with consequent migration of fluoride ions into the underlying tooth structure. Various fluorine-containing additives which have been tried in dental restorations consist of inorganic fluoride salts, organic bases such as amine hydrofluoride, fluorocarbons and fluoride-containing ion-exchange resins. These attempts to find suitable fluorine-containing additives which are both dispersed in dental restorative material and capable of reducing tooth caries through controlled long-term fluoride release have failed. Silicate cements have demonstrated cariostatic release of fluoride; however, the strictly rapid surface release of fluoride from the cement, the dissolution of the cement in oral fluids, and the low tensile strengths of the cements are major disadvantages. Alternatively, the fluoride incorporated into insoluble resin materials has been considered to be virtually incapable of leaking out and thus to be ineffective as a cariostatic agent. Studies by Forsten and Paunio (*Scandinavian Journal of Dental Research* (1972) 80, 515—519) comparing fluoride release by silicate cements and composite resins have shown that the overall release of fluoride from the two materials was comparable; however, the manner in which the fluoride was released from the composite was not controlled. Heretofore it was difficult to obtain controlled, effective cariostatic and plaque-reducing fluoride release from virtually insoluble materials such as acrylic denture base materials, adhesives and composite resins, and the like.

The use of complexes, formed by boron trifluoride in conjunction with organic amines, as curing agents is known; US—A—2824083 describes such complexes in the curing of polyepoxides. US—A—2980733 describes coordination complexes made by reacting $BF_3$ with urea and cites their uses, e.g. for fluorinating drinking water and for inclusion in dentrifrices.

In accordance with the present invention, a dental composition comprising a dental restorative material also contains a controlled fluoride release compound which comprises a complex of a Lewis base and a leachable fluorine-containing Lewis acid which is dispersible or soluble in the composition and therefore effective in preventive dentistry, wherein the fluoride release compound is present in a concentration in the range from 0.05 to 50 weight percent of the total dental composition and the restorative material is selected from composite resins, cavity liners, resin adhesives, pit and fissure sealants, denture base resins, orthodontic resins, orthodontic elastics and plastic orthodontic brackets.

The invention is based on the discovery that a controlled fluoride-release additive, comprising a Lewis base and a fluorine-containing Lewis acid, is therapeutic in preventing secondary caries and reducing plaque formation. When incorporated into polymeric dental restorative material, the additive is capable of migrating from the interior to the surface of such material, without dissolution thereof and with consequent release of fluoride.

We have now found that Lewis acid compounds containing covalently-bound fluorinde can be reacted with Lewis base compounds to produce addition compounds. When mixed with dental restorative materials, the resultant compounds in use, release fluoride ion. The fluoride is released at a controlled rate by diffusion of the Lewis acid within the dental material with subsequent hydrolysis upon contact with water. These fluorine-containing Lewis acid—Lewis base addition compounds can be added to dental restorative materials, denture base materials, orthodontic elastics, plastic dental materials and dental resins. Alternatively, the addition compounds can be incorporated into the polymer matrix as part of the monomer resin constituents. The principal requirements in respect of these compounds are that the Lewis bases are dispersible or soluble within the dental restorative material; the Lewis acids must be mobile within the restorative material and capable of migrating to the surface of such material, so as to release fluoride ion by dissociation with water at a controlled rate; and the resulting Lewis acid—Lewis base addition compound and its hydrolysis products must be non-toxic.

The Lewis acid compounds used in carrying out this invention may be any covalently-bound fluorine-containing compound with a vacant electron orbital which can be used to form a covalent bond with the electron pair of a base, yet which retains its mobility within the dental restorative material. Examples of fluorine-containing Lewis acid compounds are aluminum trifluoride, boron trifluoride, gallium trifluoride, titanium tetrafluoride and indium trifluoride. We have found that boron trifluoride works well in accordance with the present invention; thus it is the preferred fluorine-containing Lewis acid compound.

Lewis base compounds in accordance with the present invention may be any compound having an available pair of electrons, either unshared or in a π-electron orbital, which is dispersible or soluble within the dental restorative material. Preferably, the Lewis base is selected from amines, ethers, esters, acids, ketones, alcohols, mercaptans, thioethers and thioesters. In accordance with a preferred embodiment of the invention, the Lewis base is an amine represented by the formula:

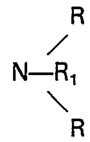

wherein R and $R_1$ are the same or different and each represents a hydrogen atom or a hydroxy group, a substituted or unsubstituted aromatic group, or a linear or branched, saturated or unsaturated, substituted or unsubstituted aliphatic group. Thus, the Lewis base compounds used in carrying out this invention can be primary amines having the formula $H_2N(R_1)$, secondary amines having the formula $HN(R_1)_2$, tertiary amines having the formula $N(R_1)_3$, ethers having the formula $R_1OR_1$, esters having the formula $R_1COOR_1$, ketones having the formula $R_1COR_1$, alcohols having the formula $R_1OH$, mercaptans, thioethers and thioesters, wherein $R_1$ is a hydroxyl, a substituted or unsubstituted aromatic group, or a linear or branched, saturated or unsaturated, substituted or unsubstituted aliphatic group, wherein the carbon chain length of these aliphatic groups is limited to the number of carbon atoms which produce a compound which is dispersible or soluble within a dental restorative material. A preferred chain length of the aliphatic group is from 1 to 18 carbon atoms. We have found that aliphatic chains containing from 1 to 12 carbon atoms operate most successfully, in accordance with this invention, and are thus most preferred. An especially preferred composition according to this invention is one in which the Lewis base is triethylamine and the Lewis acid is boron trifluoride.

Examples of Lewis base compounds useful in carrying out the present invention are: butylamine, octylamine, dodecylamine, aniline, isobutylamine, isooctylamine, isopropylamine, glycine, alanine, valine, hydroxylamine, tryptophan, aspartic acid, n - amino - 1 - butene, n - amino - 2 - octene, ethanolamine, octanolamine, dodecanolamine, 3-ethoxyaniline, dimethylamine, dibutylamine, dioctylamine, didodecylamine, methylethylamine, methylbutylamine, butyloctylamine, octyldodecylamine, methylhydrocylamine, butylethanolamine, octyldodecanolamine, methyl-isobutylamine, ethyliso-octylamine, butylisopropylamine, N-methyl-aniline, N - methyl - 3 - methoxyaniline, trimethylamine, triethylamine, trioctylamine, tridodecylamine, dimethyloctylamine, dibutyldodecylamine, triisobutylamine, triisoctylamine, triisopropylamine, dimethylisobutylamine, dibutylisoctylamine, tributeneamine, triethanolamine, triisopropanolamine, triphenylamine, methyldiphenylamine, octyldiphenylamine, pyridine, dimethylether, dibutylether, didodecylether, methylethylether, diiso:butylether, disopropylether, methylisobutylether, butylisoctylether, octylisododecylether, diphenylether and ethyl acetate.

Additional Lewis base compounds which can be used in carrying out the present invention, are aminoalkyls, for instance $R_1$ in the above formula is an aminoalkyl group of the formula:

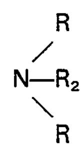

wherein each R has the meaning defined in claim 3 and $R_2$ is an alkylene group. Preferably each R in the aminoalkyl group $R_1$ is an aliphatic group having 1 to 12 carbon atoms and the alkylene group $R_2$ has 1 to 9 carbon atoms. Especially preferred Lewis base compounds in accordance with this embodiment of the invention are diamines having the formula:

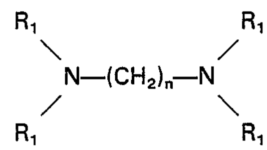

wherein each $R_1$ is as defined above and n is an integer. Thus, in the diamine formula, $R_1$ is a substituted or unsubstituted aromatic group, a hydroxyl group, hydrogen, or a linear or branched, saturated or unsaturated, substituted or unsubstituted aliphatic group having from 1 to 12 carbon atoms; n is preferably an integer from 1 to 9. The chain length of the alkylene group is limited to the number of carbon atoms which produce a compound which is dispersible or soluble within a dental restorative material. We have found that compounds having alkylene chains containing from 1 to 9 carbon atoms meet this requirement and thus are preferred in carrying out this invention. Examples of diamines which are preferred in compositions in accordance with the present invention are: N,N,N',N' - tetramethylethylenediamine, N,N,N',N' - tetraoctylbutylenediamine, N,N,N',N' - tetraisobutyloctylenediamine, N,N,N',N' - tetraphenylethylenediamine and N,N - dihydroxy - N',N' - diphenylethylenediamine. A preferred compound of the present invention is one in which the Lewis base is N,N,N',N' - tetramethylethylenediamine and the Lewis acid is boron trifluoride.

According to a preferred feature of the invention, the fluoride release compound is present in a

concentration in the range from 0.5 to 25 weight percent of the composition. Most preferably, the fluoride release compound comprises 0.5 to 1.5 weight percent of the composition.

In another embodiment of this invention, the dental restorative material is polymeric and the Lewis base is a constituent resin component thereof, i.e. the Lewis base can be monomer resin constituents capable of becoming incorporated into the backbone, side chain or crosslink of the polymeric dental material. Examples of monomer resin constituents include compounds having the formula:

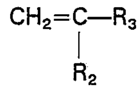

wherein $R_2$ is a hydrogen atom, a cyano group, a substituted or unsubstituted aromatic group, a linear or branched, substituted or unsubstituted, saturated or unsaturated alkyl group and $R_3$ is an esterified carboxyl group, a primary amine, a secondary amine, a tertiary amine, a carboxyl amine or an amine-containing ester having the formula:

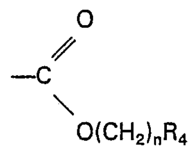

wherein $R_4$ is a primary, secondary or tertiary amine and n is an integer. Preferably, n is an integer from 1 to 18 and most preferably from 1 to 9. Examples of monomer resin constituents in accordance with this embodiment of the present invention are: acrylic and methacrylic esters, such as methylacrylate, ethylacrylate, propylacrylate, butylacrylate, 2-ethoxyhexylacrylate, methylmethacrylate, ethylmethacrylate, propylmethacrylate, butylmethacrylate, laurylmethacrylate, stearylmethacrylate, ethylisobutylacrylate, butylenedimethacrylate and trimethylolpropane trimethacrylate; acrylonitrile; trimethylaminostyrene; polyamides, such as acrylamide and methacrylamide; polycyclamide constituents, such as 1,4-cyclohexane bis(methylamine); amino resins, such as carbamide, melamine, thiocarbamide, aniline, dicyanodiamide, toluenesulphonamide, benzoguanamine, ethylene urea; acrylic acid and methacrylic acids; urethanes, epoxides, polyesters, polycarbonates and isocyanate derivatives such as toluene diisocyanate and diphenyl methane diisocyanate. A preferred Lewis base monomer resin component is diethylaminoethylmethacrylate (DEAEMA).

The present invention also consists in a method of manufacture of an orthodontic article or product, characterised in that a controlled fluoride release compound which comprises a complex of a Lewis base and a leachable fluorine-containing Lewis acid is incorporated in the article or product, which is thereby usable as a cariostatic and plaque-reducing agent.

Furthermore, the invention resides in use of a controlled fluoride release compound which comprises a complex of a Lewis base and a leachable fluorine-containing Lewis acid for the manufacture of an article or product for cariostatic and plaque-reducing dental treatments.

In carrying out the present invention, therefore, the fluorine-containing Lewis acid—Lewis base compounds are added to restorative dental materials or are incorporated into polymer-based dental restorative materials as constituent resin components. In either case, the content of the fluorine-containing compound within the dental material should be limited so as not to interfere with the physical properties of the material. In addition, the content of the fluorine-containing compound should be high enough to release fluoride ion effectively and so reduce tooth caries in a controlled sustained manner over a long period of time. Compositions containing up to about 50 wt % of the fluorine-containing Lewis acid—Lewis base addition compound do not adversely affect the physical properties of the dental materials and operate effectively as a controlled slow fluoride release composition. Thus, compositions having from 0.5 to 50 wt % of the fluorine-containing compounds are preferred. Concentrations as low as 0.05% wt of fluoride compound can be effective in preventive dentistry. A more preferred range of compositions is from 0.5 to 25 wt % of the fluorine-containing compound. The most preferred compositions in accordance with this invention contain from 0.5 to 1.5 wt % of the fluorine-containing addition compounds. It should be noted, however, that the various dental materials used in carrying out this invention will exhibit different physical characteristics when their respective formulations are modified, so that the quantity of the fluoride-containing compounds of this invention must be evaluated with each formulation.

The dental restorative materials used in accordance with this invention include resin cements, dental prosthetic devices, denture base resins, cavity liners, composite resins, pit and fissure sealants, resin adhesives, repair materials, relining and rebasing dental materials, orthodontic resins, orthodontic elastics, plastic orthodontic brackets, or any other such material used in restorative dental operations. Currently employed dental restorative materials are fabricated from polymer-based materials, metals, ceramics or combinations (composite) thereof. Examples of polymer-based materials which can thus be used in accordance with this invention include acrylic and methacrylic polymers, vinyl acrylic polymers, cyanoacrylates, polystyrene, polycarbonate, epoxy resins, nylons, vinyl styrenes, unsaturated polyesters,

polyurethane, polyvinylacetate-ethylene, silicones, polyvinylchloride, copolymer formulations using these polymers, or modifications thereof which prove useful as dental materials. Examples of dental composites which can be thus be used in accordance with this invention include glass ionomer cements, acrylic composite restorative filling materials, Bis-GMA resin composites, acrylic restorative tooth liners, and urethane dimethacrylate composites. A discussion of dental restorative materials and their composition, application, and properties can be found in *Restorative Dental Materials*, by Robert G. Craig, published by the C. V. Mosby Publishing Company, 1980. Preferred dental restorative materials in accordance with the present invention include restorative composite resins, cavity liners, adhesives, pit and fissures sealants, orthodontic resins, and denture base materials. The most preferred dental restorative materials are cavity liners, adhesives, sealants and denture base materials.

The following examples describe certain representative embodiments of this invention as set forth above. They are intended to be illustrative only and are not intended to limit the scope of the invention.

Example 1

This example describes the preparation and chemical characterization of the fluorine-containing Lewis acid—Lewis base compounds of the present invention and their use in dental restorative materials.

25 gm of diethylaminoethylmethacrylate (DEAEMA) is placed in a three-neck round-bottom flask. The flask is set into an ice bath to moderate reaction temperature. A vacuum is drawn on the reaction flask and gaseous boron trifluoride is bubbled into the DEAEMA at such a rate that the temperature does not exceed 35°C. The rate of bubbling is increased when the temperature begins to fall. The reaction is continued until the temperature no longer increases on increased boron trifluoride addition. This takes about one hour, when the reaction product is collected and then purified by distillation using a molecular still. The product prepared accordingly was determined to be the 1:1 adduct of DEAEMA and boron trifluoride (DEAEMA-$BF_3$) on the basis of infrared spectroscopy, thermogravimetric analysis, nuclear magnetic resonance spectroscopy and elemental analysis. The corresponding structure is shown below:

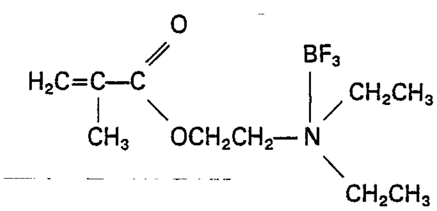

This material (DEAEMA-$BF_3$) was used in combination with other methacrylate monomers to prepare a formulation suitable for composite restorative liner preparations according to the formulation below:

35 parts Bis-GMA (reaction product of methacrylic acid and the diglycidyl ether of bisphenol A)
30 parts ethyleneglycoldimethacrylate
30 parts trimethylolpropanetrimethacrylate
5 parts DEAEMA-$BF_3$
1 part dihydroxyethyl-para-tolylamine.

The above material is polymerized by mixing 5 parts with 1 part of saturated (9%) benzoyl peroxide solution in dimethylphthalate. 0.1 gm samples of hardened material were stored in 10 ml of distilled water. The fluoride content was measured periodically with an ion-specific electrode, and significant levels of fluoride were still being released after more than three months.

Additionally, the fluoride leach (ppm/day) was determined based upon a 0.01 gm application of the composite in contact with 0.1 ml of water. For comparison, identical tests were run of films of a silico phosphate cement (Fluorothin) and a silicate cement (MQ). The results indicate that the experimental composite liner maintained a detectable release of fluoride over a longer period of time than the silicate cements, even though the silicate cements release larger amounts of fluoride during the initial part of the leach.

Example 2

This example describes another method of preparing the fluorine-containing Lewis acid—Lewis base compounds of the present invention.

37 gm of diethylaminoethylmethacrylate (DEAEMA) is placed in a three-neck round-bottom flask. Gaseous oxygen is bubbled in for one-half hour. After the oxygen flow is shut off, 26 gm of boron trifluoride methanol complex is placed in a dropping funnel. The funnel is mounted on top of the round-bottom flask in an ice water bath. The temperature is lowered to about 10°C. The boron trifluoride methanol is added dropwise while the mixture is stirred. Temperature is maintained at 15°—25°C by adjusting the addition of the boron trifluoride methanol material. Complete addition requires 20 to 30 minutes. Next, the oxygen flow is resumed to bubble off methanol and unreacted materials. After four hours, the material is collected and weighed. The yield is 49 gm.

Example 3

This example describes the preparation and physical properties of a dental adhesive in accordance with this invention.

The DEAEMA-$BF_3$ compound was prepared as in Example 1. This material was used in combination with other methacrylate monomers to prepare a formulation suitable for orthodontic adhesive preparations. Equal amounts of solutions A and B, described below, are mixed together to form the desired adhesive:

Solution A

14 parts DEAEMA-$BF_3$
28 parts diethyleneglycoldimethacrylate
29 parts Bis-GMA
27 parts fumed silica
2 parts dihydroxyethyl-para-tolylamine.

Solution B

42 parts diethyleneglycoldimethacrylate
29 Bis-GMA
27 parts fumed silica
1 part benzoyl peroxide.

Fluoride release

Cured discs of material measuring .015 to 0.050 inches (0.4 to 1.27 mm) thick and 1.5 inches (38 mm) in diameter were immersed in 50 ml portions of distilled water. The fluoride concentration of the exposed water was determined at measured intervals using a fluoro-specific ion electrode. After each determination, the water was discarded and replaced with an additional portion of distilled water. Rates of release have been determined to be 2 to 5 micrograms of fluoride per square centimeter per day. The samples thus studied have fluoride available for release for over one year.

Example 4

The DEAEMA-$BF_3$ adduct is prepared according to Example 1, and is used in combination with other methacrylate monomers to prepare a formulation suitable for denture-base preparations according to the following composition: The liquid resin portion of a powder or liquid denture-base material is formulated by mixing 1 part of a solution containing 4 parts DEAEMA-$BF_3$ and 6 parts methylmethacrylate containing 2% dimethyl-para-toluidine and 2 parts commercial denture-base powder (polymethylmethacrylate) to form test specimens and acrylic dental devices.

Example 5

The DEAEMA-$BF_3$ addition compound can be prepared according to Example 1 and used in combination with other methacrylate monomers to prepare a formulation suitable for pit and fissure sealant preparations according to the following composition: Equal amounts of solution A and solution B as described below can be mixed together to form a chemically cured pit and fissure sealant:

Solution A

20 parts DEAEMA-$BF_3$
40 parts diethyleneglycoldimethacrylate
40 parts Bis-GMA
2 parts dihydroxyethyl-para-tolylamine.

Solution B

60 parts diethyleneglycoldimethacrylate
40 parts Bis-GMA
1 part benzoyl peroxide.

Example 6

The DEAEMA-$BF_3$ addition compound was prepared as shown in Example 1 and used in combination with other methacrylate monomers to prepare a formulation suitable for composite restorative liner preparations according to the following composition:

35 parts Bis-GMA
30 parts ethyleneglycoldimethacrylate
30 parts trimethylolpropanetrimethacrylate
2.5 parts DEAEMA-$BF_3$
1 part dihydroxyethyl-para-tolylamine.

The above material is polymerized by mixing 5 parts with 1 part of saturated 9% benzoyl peroxide solution in dimethylphthalate. 0.1 gm samples of hardened material were stored in 10 ml of distilled water. The fluoride content was measured periodically with an ion-specific electrode and significant levels of fluoride were still being released after more than three months. For comparison, identical tests were run of films of fluorothin, a silica phosphate cement, and MQ, a silicate cement. Results indicate that the experimental composite films maintained a detectable release of fluoride over a longer period of time than the fluorothin and MQ films, even though the silica phosphate and silicate cements released larger amounts of fluoride during the initial part of the leach.

Example 7

15 gm of N,N,N',N' - tetramethylethylenediamine (TMED) is placed in a three-neck round-bottom flask. The flask is set into an ice bath to moderate reaction temperature. A vacuum is drawn on the reaction flask and gaseous boron trifluoride is bubbled at such a rate that the temperature does not exceed 35°C. The rate of bubbling is increased when the temperature begins to fall. The reaction is continued until the temperature no longer increases on increased boron trifluoride addition. This takes about one hour, when the reaction product is collected and then purified by distillation using a molecular still.

The resulting addition compound is used in combination with methacrylate monomers to prepare a formulation suitable for composite resin filling materials according to the formulation below:

13 parts Bis-GMA
3 parts trimethylolpropanetrimethacrylate
3 parts ethyleneglycoldimethacrylate
.01 parts 2,6-di-t-butyl-p-cresol
.2 parts N,N-diethanol-m-tolylamine
1 part permasorb MA
17 parts calcium silicate
.04 parts tilanium dioxide
60 parts barium silicate
3 parts TMED $BF_3$.

Example 8

The DEAEMA-$BF_3$ compound was prepared as shown in Example 1. This compound was then added to an experimental formulation of a composite restorative liner as shown below:

35 parts Bis-GMA
30 parts ethyleneglycoldimethacrylate
30 parts trimethylolpropanetrimethacrylate
5 parts DEAEMA-$BF_3$
0.84 pphr N,N-diethanol-p-tolylamine
0.05 pphr 2,6-di-t-butyl-p-cresol
2.5 pphr benzophenone.

Results of tests show that the thermal and ultraviolet color stabilities of these liners are good, as is their compatibility with silicate cements and composite resins.

Example 9

A composite restorative liner was prepared as shown in Example 6. Analysis of leaching of the fluorine-containing Lewis acid—Lewis base addition compound indicates that the boron content of the restorative liner leaching solution is much less than it should be if all the available boron is leached.

| Solution (% adduct) | Leach sol'n fluoride content | % Total avail. fluoride leached | Avail. boron* | Exp. boron in leach sol'n | % of Boron avail. |
|---|---|---|---|---|---|
| (5%) | 12 μg | 22.5 | 40 μg | 7.4 μg | 18.5% |
| (2.5%) | 8.4 μg | 32 | 29 μg | 2.6 μg | 8.8%* |

* Based upon measured fluoride content in leach solution.

To measure the fluoride-releasing characteristics of cured experimental restorative liners, 0.1 gm films of 9 to 13 mils (0.23 to 0.33 mm) thickness were soaked for an extended period in 10 mls of water. Fluoride ion concentrations were measured periodically, using an ion-specific electrode.

**Claims**

1. A dental composition comprising a dental restorative material, characterised in that the composition also contains a controlled fluoride release compound which comprises a complex of a Lewis base and a leachable fluorine-containing Lewis acid which is dispersible or soluble in the composition and therefore effective in preventive dentistry, wherein the fluoride release compound is present in a concentration in the range from 0.05 to 50 weight percent of the total dental composition and the restorative material is selected from composite resins, cavity liners, resin adhesives, pit and fissure sealants, denture base resins, orthodontic resins, orthodontic elastics and plastic orthodontic brackets.

2. A composition according to claim 1, wherein the Lewis base is selected from amines, ethers, esters, acids, ketones, alcohols, mercaptans, thioethers and thioesters.

3. A composition according to claim 2, wherein the Lewis base is an amine represented by the formula:

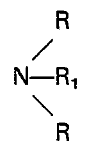

wherein R and $R_1$ are the same or different and each represents a hydrogen atom or a hydroxy group, a substituted or unsubstituted aromatic group, or a linear or branched, saturated or unsaturated, substituted or unsubstituted aliphatic group.

4. A composition according to claim 3, wherein each aliphatic group has from 1 to 12 carbon atoms.

5. A composition according to claim 3 or 4, wherein the Lewis base is triethylamine and the Lewis acid is boron trifluoride.

6. A composition according to claim 3, wherein $R_1$ is an aminoalkyl group of the formula:

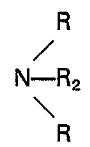

wherein each R has the meaning defined in claim 3 and $R_2$ is an alkylene group.

7. A composition according to claim 6, wherein each R in the aminoalkyl group $R_1$ is an aliphatic group having 1 to 12 carbon atoms and the alkylene group $R_2$ has 1 to 9 carbon atoms.

8. A composition according to any of claims 3 to 7, wherein the Lewis base is a diamine represented by the formula:

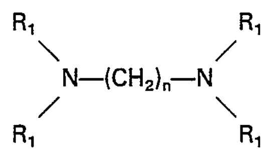

wherein each $R_1$ has the meaning defined in claim 3 and n is an integer.

9. A composition according to claim 8, wherein the Lewis base is N,N,N',N' - tetramethylethylenediamine and the Lewis acid is boron trifluoride.

10. A composition according to any preceding claim, wherein the fluoride release compound is present in a concentration in the range from 0.5 to 25 weight percent of the composition.

11. A composition according to claim 10, wherein the fluoride release compound comprises 0.5 to 1.5 weight percent of the composition.

12. A composition according to any preceding claim, wherein the dental restorative material is polymeric and the Lewis base is a constituent resin component thereof.

13. A composition according to claim 12, wherein the Lewis base constituent resin component has the formula:

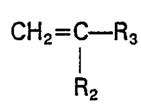

wherein $R_2$ is a hydrogen atom, a cyano group, a substituted or unsubstituted aromatic group, a linear or branched, substituted or unsubstituted, saturated or unsaturated alkyl group and $R_3$ is an esterified carboxyl group, a primary amine, a secondary amine, a tertiary amine, a carboxyl amine or an amine-containing ester having the formula:

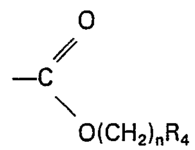

wherein $R_4$ is a primary, secondary or tertiary amine and n is an integer.

14. A composition according to claim 13, wehrein n is from 1 to 9.

15. A composition according to claim 13, wherein the amine is a tertiary amine of the formula:

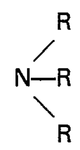

wherein R is a substituted or unsubstituted aromatic group or a linear or branched, saturated or unsaturated, substituted or unsubstituted aliphatic group.

16. A composition according to claim 12, wherein the Lewis base is selected from acrylic and methacrylic esters, polyamides, polycyclamides, amino resins, acrylic and methacrylic acids, urethanes, epoxides, polyesters and polycarbonates.

17. A composition according to any of claims 13 to 16, wherein the constituent resin component is dimethylaminoethylmethacrylate.

18. An orthodontic article of product, for use as a cariostatic and plaque-reducing agent in preventive dentistry, characterised in that it incorporates a controlled fluoride release compound which comprises a complex of a Lewis base and a leachable fluorine-containing Lewis acid.

19. An orthodontic article or product according to claim 18, which includes a dental composition as defined in any of claims 1 to 17.

20. An orthodontic article or product according to claim 18 or 19, in the form of a resin, an elastic or a plastic bracket.

21. A method of manufacture of an orthodontic article or product, characterised in that a controlled fluoride release compound which comprises a complex of a Lewis base and a leachable fluorine-containing Lewis acid is incorporated in the article or product, which is thereby usable as a cariostatic and plaque-reducing agent.

22. A method according to claim 21, wherein the controlled fluoride release compound is a dental composition as defined in any of claims 1 to 17.

23. Use of a controlled fluoride release compound which comprises a complex of a Lewis base and a leachable fluorine-containing Lewis acid for the manufacture of an article or product for cariostatic and plaque-reducing dental treatments.

**Patentansprüche**

1. Zahnärztliche Masse, bestehend aus einem zahnheilkundlich wirksamen Material, dadurch gekennzeichnet, daß die Masse auch eine, eine kontrollierte Fluoridfreisetzung ermöglichende Verbindung enthält, die aus einer Lewisbase und einer auslaugbaren, fluorenthaltenden Lewissäure besteht, daß die letztgenannte Verbindung in der Masse dispergierbar oder lösbar ist und demzufolge im Rahmen der präventiven Zahnheilkunde einsetztbar ist, daß die eine Fluoridfreisetzung ermöglichende Verbindung bezogen auf die genannte Masse in einer Konzentration von 0,05 bis 50 Gew.-% vorliegt und daß das zahnheilkundlich wirksame Material aus Harzmischungen, Hohlraumeinlagen, Harzklebstoffen, Versiegelungsmitteln für Löcher und Spalte, Basisharze für Zahnersatz, orthodonte Harze, orthodonte elastische Stoffe oder plastischen orthodonten Stützmitteln besteht.

2. Zahnärztliche Masse nach Anspruch 1, dadurch gekennzeichnet, daß die Lewisbase aus Aminen, Äthern, Estern, Säuren, Ketonen, Alkoholen, Merkaptanen, Thioäthern oder Thioestern besteht.

3. Zahnärztliche Masse nach Anspruch 2, dadurch gekennzeichnet, daß die Lewisbase durch ein, durch die folgende Formel darstellbares Amin gebildet wird

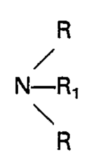

wobei R und $R_1$ einander gleich oder voneinander verschieden sind und jeder ein Wasserstoffatom oder eine Hydroxydgruppe, eine substituierte oder unsubstituierte aromatische Gruppe, eine lineare oder verzweigte, substituierte oder unsubstituierte aliphatische Gruppe darstellen.

4. Zahnärztliche Masse nach Anspruch 3, dadurch gekennzeichnet, daß jede aliphatische Gruppe 1 bis 12 Kohlenstoffatome aufweist.

5. Zahnärztliche Masse nach Anspruch 3 oder 4, dadurch gekennzeichnet, daß die Lewisbase aus Triäthylamin und die Lewissäure aus Bortrifluorid besteht.

6. Zahnärztliche Masse nach Anspruch 3, dadurch gekennzeichnet, daß $R_1$ ein Aminoalkyl der nachstehenden Formel ist

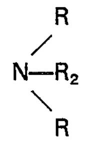

wobei jedem R die in Anspruch 3 erwähnte Bedeutung zukommt und wobei $R_2$ eine Alkengruppe ist.

7. Zahnärztliche Masse nach Anspruch 6, dadurch gekennzeichnet, daß jedes R in der Aminoalkylgruppe $R_1$ eine aliphatische Gruppe mit 1 bis 12 Kohlenstoffatomen ist und daß die Alkengruppe $R_2$ 1 bis 9 Kohlenstoffatome aufweist.

8. Zahnärztliche Masse nach einem der vorangegangenen Ansprüche 3 bis 7, dadurch gekennzeichnet, daß die Lewisbase ein durch die folgende Formel darstellbares Diamin ist

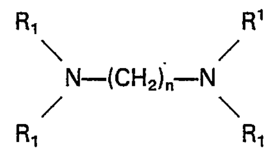

wobei $R_1$ die im Anspruch 3 erwähnte Bedeutung hat und wobei n eine ganze Zahl ist.

9. Zahnärztliche Masse nach Anspruch 8, dadurch gekennzeichnet, daß die Lewisbase ein N,N,N',N' - Tetramethyläthylendiamin und die Lewissäure ein Bortrifluorid ist.

10. Zahnärztliche Masse nach einem der vorangegangenen Ansprüche, dadurch gekennzeichnet, daß die die Fluoridfeisetzung ermöglichende Verbindung bezogen auf die genannte Masse in einer Konzentration von 0,5 bis 25 Gew.-% vorliegt.

11. Zahnärztliche Masse nach Anspruch 10, dadurch gekennzeichnet, daß die die Fluoridfreisetzung ermöglichende Verbindung bezogen auf die genannten Masse in einer Konzentration von 0,5 bis 1,5 Gew.-% vorliegt.

12. Zahnärztliche Masse nach einem der vorangegangenen Ansprüche, dadurch gekennzeichnet, daß das zahnheilkundlich wirksame Material ein polymerer Stoff ist und die Lewisbase ein Harzbestandteil desselben ist.

13. Zahnärztliche Masse nach Anspruch 12, dadurch gekennzeichnet, daß die einen Harzbestandteil der Lewisbase bildende Komponente durch die folgende Formel darstellbar ist

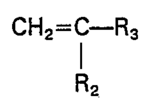

worin $R_2$ ein Wasserstoffatom, eine Zyangruppe, eine substituierte oder unsubstituierte aromatische Gruppe, eine lineare oder verzweigte, substituierte oder unsubstituierte, gesättigte oder ungesättigte Alkylgruppe und worin $R_3$ eine veresterte Karboxylgruppe, ein primäres, sekundäres oder te tiäres Amin oder ein aminhaltiger Ester der folgenden Formel ist

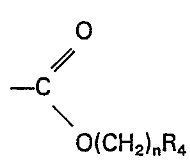

worin $R_4$ ein primäres sekundäres oder tertiäres Amin und n eine ganze Zahl bedeuten.

14. Zahnärztliche Masse nach Anspruch 13, dadurch gekennzeichnet, daß n eine Zahl zwischen 1 und 9 ist.

15. Zahnärztliche Masse nach Anspruch 13, dadurch gekennzeichnet, daß die tertiären Amine durch die folgende Formel darstellbar sind

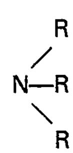

worin R eine substituierte oder unsubstituierte aromatische Gruppe oder eine lineare oder verzweigte, gesättigte oder ungesättigte, substituierte oder unsubstituierte aliphatische Gruppe ist.

16. Zahnärztliche Masse nach Anspruch 12, dadurch gekennzeichnet, daß die Lewisbase aus Acryl oder Metacryl, Estern, Polyamiden, polyzyklischen Amiden, Aminharzen, Acryl- und Metacrylsäuren, Urethanen, Epoxiden, Polyestern oder Polykarbonaten besteht.

17. Zahnärztliche Masse nach einem der vorangegangenen Ansprüche 13 bis 16, dadurch gekennzeichnet, daß die einen Harzbestandteil bildende Komponente aus Dimethylamináthylmetacrylat besteht.

18. Orthodonte Artikel oder Produkt zur Anwendung in der präventiven Zahnheilkunde als karieshemmendes und Zahnbelag reduzierendes Mittel, dadurch gekennzeichnet, daß es eine, eine kontrollierte Fluoridfreisetzung ermöglichende Verbindung umfaßt, die aus einem Komplex aus einer Lewisbase und einer auslaugbaren fluorenthaltenden Lewissäure besteht.

19. Orthodonter Artikel nach Anspruch 18, dadurch gekennzeichnet, daß er eine zahnärztliche Masse entsprechend einem der vorangegangenen Ansprüche 1 bis 17 mit umfaßt.

20. Orthodonter Artikel nach Anspruch 18 oder 19, dadurch gekennzeichnet, daß es sich um Harz, ein elastisches oder ein plastisches Stützmittel handelt.

21. Verfahren zur Herstellung eines orthodonten Artikels bzw. Produktes, dadurch gekennzeichnet, daß eine, eine kontrollierte Fluoridfreisetzung ermöglichende Verbindung, die aus einem Komplex aus einer Lewisbase und einer auslaugbaren, fluorenthaltenden Lewissäure besteht, in den Artikel bzw. das Produkt eingebunden wird, so daß letzteres als karieshemmendes und Zahnbelag reduzierendes Mittel benutzbar ist.

22. Verfahren nach Anspruch 21, dadurch gekennzeichnet, daß die eine kontrollierte Fluoridfreisetzung ermöglichende Verbindung eine zahnärztliche Masse entsprechend den Ansprüchen 1 bis 17 ist.

23. Verwendung einer eine kontrollierte Fluoridfreisetzung ermöglichenden Verbindung, welche aus einem Komplex, bestehend aus einer Lewisbase und einer auslaugbaren, fluorenthaltenden Lewissäure besteht, zur Herstellung eines Artikels oder Produktes für karieshemmende und Zahnbelag reduzierende zahnärztliche Behandlungen.

**Revendications**

1. Composition dentaire, comprenant une matière de reconstitution dentaire, caractérisée en ce qu'elle contient aussi un composé capable de libération contrôlée de fluorure qui est constitué par un complexe d'une base de Lewis et d'un acide de Lewis fluoré lixiviable et qui est dispersable ou soluble dans la composition et, par conséquent, efficace en dentisterie préventive, le composé capable de libération de fluorure étant présent en une concentration dans la gamme de 0,05 à 50% en pods de la composition dentaire totale et la matière de reconstitution étant choisie entre des résines composites, des produits d'obturation des cavités, des résines adhésives, des produits de bouchage des piqûres et fissures, des résines de base des dentiers, des résines d'orthodontie, des élastique d'orthodontie et des supports d'orthodontie en matière plastique.

2. Composition selon la revendication 1, caractérisée en ce que la base de Lewis est choisie entre des amines, des éthers, des esters, des acides, des cétones, des alcools, des mercaptans, des thio-éthers et des thio-esters.

3. Composition selon la revendication 2, caractérisée en ce que la base de Lewis est une amine représentée par la formule

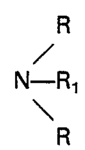

dans laquelle R et $R_1$ sont identiques ou différents et rperésentent chacun un atome d'hydrogène ou un groupe hydroxy, un groupe aromatique substitué ou non substitué ou un groupe aliphatique à chaîne droite ou ramifiée, saturé ou insaturé, substitué ou non substitué.

4. Composition selon la revendication 3, caractérisée en ce que chaque groupe aliphatique comporte de 1 à 12 atomes de carbone.

5. Composition selon la revendication 3 ou 4, caractérisée en ce que la base de Lewis est la triéthylamine et l'acide de Lewis est le trifluorure de bore.

6. Composition selon la revendication 3, caractérisée en ce que $R_1$ est un groupe amino-alkyle de formule

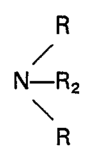

dans laquelle chaque symbole R a la signification donnée dans la revendication 3 et $R_2$ est un groupe alkylène.

7. Composition selon la revendication 6, caractérisée en ce que R dans le groupe amino-alkyle $R_1$ est un groupe aliphatique à 1—12 atomes de carbone et le groupe alkylène $R_2$ comporte 1 à 9 atomes de carbone.

8. Composition selon l'une quelconque des revendications 3 à 7, caractérisée en ce que la base de Lewis est une diamine de formule

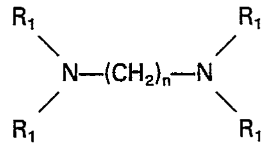

dans laquelle chaque symbole $R_1$ a la signification donnée dans la revendication 3 et n est un nombre entier.

9. Composition selon la revendication 8, caractérisée en ce que la base de Lewis est la N,N,N',N' - tétraméthyléthylènediamine et l'acide de Lewis est le trifluorure de bore.

10. Composition selon l'une quelconque des revendications 1 à 9, caractérisée en ce que le composé capable de libération de fluorure est présent en une concentration dans la gamme de 0,5 à 25% en poids de la composition.

11. Composition selon la revendication 10, caractérisée en ce que le composé capable de libération de fluorure entre pour 0,5 à 1,5% en poids dans la constitution de la composition.

12. Composition selon l'une quelconque des revendications 1 à 11, caractérisée en ce que la matière de reconstitution dentaire est de nature polymère et la base de Lewis est un élément constitutif de résine de cette matière.

13. Composition selon la revendication 12, caractérisée en ce que l'élément constitutif de résine formant la base de Lewis répond à la formule

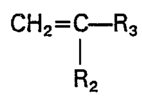

dans laquelle $R_2$ est un atome d'hydrogène, un groupe cyano, un groupe aromatique substitué ou non substitué, un groupe alkyle à chaîne droite ou ramifiée, substitué ou non substitué, saturé ou insaturé, et $R_3$ est un groupe carboxyle estérifié, amine primaire, amine secondaire, amine tertiaire, carboxylamine ou ester aminé de formule

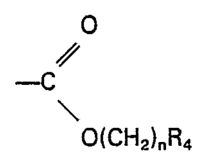

dans laquelle $R_4$ est une amine primaire, secondaire ou tertiaire et n est un nombre entier.

14. Composition selon la revendication 13, caractérisée en ce que n est compris entre 1 et 9.

15. Composition selon la revendication 13, caractérisée en ce que l'amine est une amine tertiaire de formule

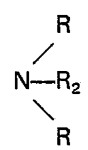

dans laquelle R est un groupe aromatique substitué ou non substitué ou un groupe aliphatique à chaîne droite ou ramifiée, saturé ou insaturé, substitué ou non substitué.

16. Composition selon la revendication 12, caractérisée en ce que la base de Lewis est choisie entre des esters acryliques et méthacryliques, des polyamides, des polycyclamides, des résines aminiques, les acides acrylique et méthacrylique, des uréthanes, des époxydes, des polyesters et des polycarbonates.

17. Composition selon l'une quelconque des revendications 13 à 16, caractérisée en ce que l'élément constitutif de résine est le méthacrylate de diméthylaminoéthyle.

18. Article ou produit d'orthodontie, utilisable comme agent cariostatique ou réducteur de la plaque en dentisterie préventive, caractérisé en ce qu'il y est incorporé un composé capable de libération contrôlée de fluorure qui est constitué par un complexe d'une base de Lewis et d'un acide de Lewis fluoré lixiviable.

19. Article ou produit d'orthodontie selon la revendication 18, caractérisé en ce qu'il contient une composition dentaire selon l'une quelconque des revendications 1 à 17.

20. Article ou produit d'orthodontie selon la revendication 18 ou 19, sous la forme d'une résine, d'un corps élastique ou d'un support en matière plastique.

21. Procédé de fabrication d'un article ou produit d'orthodontie, caractérisé en ce qu'un composé capable de libération contrôlée de fluorure, qui est constitué par un complexe d'une base de Lewis et d'un acide de Lewis fluoré lixiviable, est incorporé dans l'article ou produit, qui est ainsi utilisable comme agent cariostatique ou réducteur de la plaque.

22. Procédé selon la revendication 21, caractérisé en ce que le composé capable de libération contrôlée de fluorure est une composition dentaire selon l'une quelconque des revendications 1 à 17.

23. Utilisation d'un composé capable de libération contrôlée de fluorure, qui est constitué par un complexe d'une base de Lewis et d'un acide de Lewis fluoré lixiviable, pour la fabrication d'un article ou produit pour des traitements cariostatiques ou de réduction de la plaque en dentisterie.